# EUROPÄISCHE PATENTSCHRIFT

(11) **EP 0 481 318 B1**
(45) Veröffentlichungstag und Bekanntmachung des Hinweises auf die Patenterteilung: **07.06.1995**
(21) Anmeldenummer: 91117030.6
(22) Anmeldetag: 07.10.1991
(51) Int. Cl.: C07D 229/00, C08G 18/79

(54) **Verfahren zur Herstellung von Uretdion- und/oder Isocyanuratgruppen aufweisenden Polyisocyanaten**
Process for the preparation of polyisocyanates containing urethdione and isocyanate groups
Procédé de préparation de polyisocyanates portant des groupes d'urétrolinedione et d'isocyanate

(30) Priorität: 19.10.1990 DE 4033288
(43) Veröffentlichungstag der Anmeldung: 22.04.1992
(73) Patentinhaber: BAYER AG, 51368 Leverkusen (DE)
(72) Erfinder: Laas, Hans Josef, Dr., W-5000 Köln 60 (DE); Halpaap, Reinhard, Dr., W-5068 Odenthal (DE); Pedain, Josef, Dr., W-5000 Köln 80 (DE)

(56) Entgegenhaltungen:
- EP-A- 0 377 177
- DE-A- 1 670 720
- DE-A- 1 954 093
- DE-A- 3 437 635
- DE-A- 3 900 053
- US-A- 4 614 785

## Beschreibung

Die Erfindung betrifft ein neues Verfahren zur Herstellung von Uretdion- und/oder Isocyanuratgruppen aufweisenden Polyisocyanaten durch Dimerisierung und/oder Trimerisierung (Oberbegriff: Oligomerisierung) eines Teils der Isocyanatgruppen von organischen Diisocyanaten unter Verwendung von dreiwertigen Phosphor enthaltenden Katalysatoren und Abbruch der Oligomerisierungsreaktion unter oxidativer Desaktivierung des Katalysators.

Die Oligomerisierung von organischen Diisocyanaten unter Verwendung von dreiwertigen Phosphor enthaltenden Katalysatoren, insbesondere von tertiären Phosphinen oder peralkylierten Phosphorigsäuretriamiden ist aus einer Reihe von Veröffentlichungen bekannt. So lassen sich aus Mischungen aromatischer und aliphatischer Diisocyanate nach dem Verfahren der DE-OS 1 670 667 oder DE-OS 1 954 093 unter Einwirkung von tertiären Phosphinen die den Diisocyanaten entsprechenden Isocyanuratgruppen aufweisenden Polyisocyanate herstellen. Aus den DE-OS'en 1 670 720, 1 934 763, 3 900 053 oder der US-PS 4 614 785 ist ferner bekannt, daß die gleichen Katalysatoren auch die Dimerisierung von aliphatischen Diisocyanaten unter Bildung von Uretdionstrukturen beschleunigen. Reine Dimerisate aliphatischer Diisocyanate lassen sich nach den DE-OS'en 3 080 513, 3 227 779 und 3 437 635 durch Katalyse mit peralkylierten Phosphorigsäuretriamiden, gegebenenfalls in Gegenwart von H-aciden Co-Katalysatoren, wie in der letztgenannten Veröffentlichung beschrieben, gewinnen.

Zur Herstellung von Produkten mit definiert reproduzierbaren Eigenschaften ist es in allen Fällen erforderlich, die Oligomerisierungsreaktion genau an einem vorbestimmten Punkt abzubrechen.

Nach den Verfahren der DE-OS'en 3 030 513 und 3 227 779 wird dazu beim gewünschten Oligomerisierungsgrad der Katalysator gemeinsam mit überschüssigem, nicht umgesetztem Diisocyanat abdestilliert. Diese Vorgehensweise hat jedoch den Nachteil, daß es insbesondere aufgrund der hohen Temperaturen während der destillativen Aufarbeitung zu unkontrolliert ablaufenden Nebenreaktionen kommen kann. Desweiteren muß das Destillat, das den aktiven Katalysator enthält und somit nur bedingt lagerstabil ist, sofort weiterverarbeitet werden.

Zur Umgehung dieser Schwierigkeiten wird bei den meisten der oben beschriebenen Verfahren des Standes der Technik die Oligomerisierungsreaktion bei einem bestimmten Oligomerisierungsgrad durch Desaktivierung des Katalysators abgebrochen, bevor das überschüssige, nicht umgesetzte Diisocyanat destillativ von Harz getrennt wird. Diese Desaktivierung erfolgt im allgemeinen durch Zugabe eines Katalysatorgifts, wobei in den einschlägigen Vorveröffentlichungen als Katalysatorgift insbesondere Verbindungen vorgeschlagen werden, die mit den Katalysatoren unter Salzbildung reagieren. Dazu gehören Alkylierungsmittel, wie Methyliodid, Dimethylsulfat und Toluolsulfonsäuremethylester (DE-OS'en 1 670 667, 1 670 720), Acylierungsmittel wie Benzoylchlorid, Acetylchlorid, Acetanhydrid, Bernsteinsäureanhydrid und Chlorameisensäureester (DE-OS'en 1 670 667, 1 670 720, 1 934 763), Säuren wie Chloressigsäure, Trichloressigsäure, Trifluoressigsäure, Methansulfonsäure, Perfluorbutansulfonsäure, Phosphorsäure, saure Phosphorsäureester, gasförmiger Chlorwasserstoff sowie Chlorwasserstoff abspaltende Verbindungen wie Carbamidsäurechloride (DE-OS'en 1 670 667, 3 437 635). Außerdem eignen sich beispielsweise gemäß US-PS 4 614 785 Sulfonylisocyanate bzw. gemäß DE-OS 1 954 093 elementarer Schwefel zur Desaktivierung von Phosphinkatalysatoren.

Bei Verwendung solcher Katalysatorgifte treten in der Praxis jedoch eine Reihe von Schwierigkeiten auf.

Die Desaktivierung des Katalysators soll nach den oben geschilderten Verfahren des Standes der Technik mit einer äquimolaren Menge an Abstopper vorgenommen werden. Da während der Modifizierungsreaktion jedoch immer eine mehr oder weniger große Menge des Katalysators verbraucht wird, ist es meist schwierig, die exakt benötigte, d.h. äquimolare Menge an Katalysatorgift zu bestimmen. Bei Verwendung zu geringer Menge an Abstopper bleibt aber ein Teil des Katalysators aktiv, was während und nach der destillativen Aufarbeitung zu den gleichen Problemen führen kann, wie sie bei den oben geschilderten Verfahren ohne Reaktionsstoppung auftreten. Bei Überabstoppung, d.h. bei Einsatz zu großer Mengen an Abstopper, kann der eingesetzte Überschuß während der Abtrennung nicht umgesetzten Diisocyanates ins Destillat gelangen, welches sich dann bei Rückführung zur erneuten Verwendung nur noch schwer oder überhaupt nicht mehr aktivieren läßt.

Das aus Katalysator und Katalysatorgift gebildete Reaktionsprodukt sollte einen höheren Siedepunkt besitzen als die eingesetzten monomeren Diisocyanate, sich unter den Bedingungen der destillativen Aufarbeitung nicht zersetzen und somit im Harz verbleiben. Dies ist bei der Verwendung salzbildender Katalysatorgifte im allgemeinen der Fall. Die bei der Desaktivierung von Phosphinkatalysatoren mit elementarem Schwefel (DOS 1 954 093) entstehenden Phosphinsulfide sind jedoch unter den zur Destillation benötigten Bedingungen leichtflüchtig, so daß sie sich, insbesondere bei mehrfacher Recyclisierung des Destillats, als Verunreinigungen in diesem anreichern.

Einige der beschriebenen Katalysatorgifte, so z.B. Sulfonylisocyanate nach der US-PS 4 614 785, üben außerdem einen negativen Einfluß auf die Farbqualität der gebildeten Polyisocyanate aus.

Es war daher die der Erfindung zugrundeliegende Aufgabe, ein neues Verfahren zur Oligomerisierung von Diisocyanaten unter Verwendung der an sich bekannten Dimerisierungs- und/oder Trimerisierungskatalystoren zur Verfügung zu stellen, das nicht mit den geschilderten Schwierigkeiten des Standes der Technik behaftet ist. Das Verfahren sollte eine Desaktivierung des Katalysators derart ermöglichen, daß nach destillativer Abtrennung überschüssiger, monomerer Diisocyanate eine völlige Lagerstabilität von Polyisocyanat und Destillat gewährleistet ist. Die Reaktivität des Harzes gegenüber NCO-reaktiven Gruppen soll außerdem erhalten bleiben, das Destillat frei von Verunreinigungen und somit recyclisierbar sein, wobei außerdem durch die Desaktivierung des Katalysators keine Verfärbungen oder Trübungen im Verfahrensprodukt auftreten dürfen.

Diese Aufgabe konnte überraschenderweise mit dem nachstehend näher beschriebenen erfindungsgemäßen Verfahren gelöst werden, Das erfindungsgemäße Verfahren beruht auf dem Prinzip, die eingesetzten dreiwertigen Phosphor enthaltenden Katalysatoren, insbesondere tertiären Phosphine durch geeignete Oxidationsmittel in 5-wertige, katalytisch unwirksame Derivate, insbesondere in die entsprechenden Phosphinoxide zu überführen.

Gegenstand der Erfindung ist ein Verfahren zur Herstellung von Uretdion- und/oder Isocyanuratgruppen aufweisenden Polyisocyanaten durch Oligomerisierung eines Teils der Isocyanatgruppen von organischen Diisocyanaten unter Bildung von Uretdion- und/oder Isocyanuratgruppen in Gegenwart von dreiwertigen Phosphor enthaltenden Katalysatoren, Abbruch der Oligomerisierungsreaktion bei einem gewünschten Oligomerisierungsgrad und anschließende Entfernung des nicht umgesetzten Diisocyanatüberschusses durch Dünnschichtdestillation, dadurch gekennzeichnet, daß man die Reaktion mit Hilfe eines geeigneten Oxidationsmittels unter Umwandlung des Katalysators in eine katalytisch inaktive, oxidierte Form abbricht.

Ausgangsmaterialien für das erfindungsgemäße Verfahren sind beliebige organische Diisocyanate, wie z.B. aliphatische, cycloaliphatische, araliphatische oder aromatische Diisocyanate des Molekulargewichtsbereichs 140 bis 400 oder beliebige Gemische solcher Diisocyanate.

Beispielhaft seien hier genannt 1,4-Diisocyanatobutan, 1,6-Diisocyanatohexan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanato-cyclohexan, 1-Isocyanato-3,3,5-trimethyl-5-isocyanatomethylcyclohexan (Isophorondiisocyanat), 1,3- und 1,4-Xylylendiisocyanat, 4,4-Diisocyanatodicyclohexylmethan, 1,3- und/oder 1,4-Bis-(2-isocyanato-prop-2-yl)-benzol, 1-Isocyanato-1-methyl-4(3)-isocyanatomethylcyclohexan, 4-(4'-Methyl-3'-isocyanatobenzyl)-cyclohexylisocyanat, 2,4- und 2,6-Diisocyanatotoluol, 2,4'- und 4,4'-Diisocyanatodiphenylmethan, oder 1,5-Diisocyanatonaphthalin,

Bevorzugt werden beim erfindungsgemäßen Verfahren Diisocyanate mit 6 bis 15 C-Atomen mit (cyclo)aliphatisch gebundenen Isocyanatgruppen als Ausgangsmaterialien eingesetzt. Besonders bevorzugt ist die Verwendung von 1,6-Diisocyanatohexan (HDI) und Isophorondiisocyanat (IPDI) als erfindungsgemäße Ausgangsmaterialien. Selbstverständlich können auch Gemische der genannten Diisocyanate verwendet werden.

Geeignete Katalysatoren für das erfindungsgemäße Verfahren sind beliebige, dreiwertigen Phosphor enthaltende organische Verbindungen, insbesondere tertiäre organische Phosphine oder peralkylierte Phosphorigsäuretriamide oder deren Gemische, wie sie in der US-PS 46 14 785, Kolonne 4, Zeile 11 bis Kolonne 5, Zeile 5 beispielhaft beschrieben sind. Für das erfindungsgemäße Verfahren bevorzugte Katalysatoren sind solche, deren oxidierte Form einen höheren Siedepunkt aufweist als die eingesetzten monomeren Diisocyanate.

Bei Verwendung von 1,6-Diisocyanatohexan und/oder Isophorondiisocyanat als Ausgangsdiisocyanat beim erfindungsgemäßen Verfahren ist daher Tri-n-octylphosphin der besonders bevorzugte Katalysator.

Die erfindungsgemäßen Katalysatoren werden i.a. in Mengen von 0,01 bis 5 Gew.-%, vorzugsweise 0,1 bis 2 Gew,-%, bezogen auf die Menge des eingesetzten Ausgangsdiisocyanats, eingesetzt.

Als Co-Katalysatoren können beim erfindungsgemäßen Verfahren gegebenenfalls beliebige organische Verbindungen, die mindestens ein an Sauerstoff, Stickstoff oder Schwefel gebundenes Wasserstoff-Atom und einen pKₛ von mindestens 6 aufweisen, mitverwendet werden, wie sie in der DE-OS 34 37 635, Seite 11, Zeile 8 bis Seite 16, Zeile 6, beschrieben sind.

Als Co-Katalysatoren werden niedermolekulare, ein- oder mehrwertige Alkohole, d.h. insbesondere solche des Molekulargewichtsbereichs 32 bis 200 bzw. beliebige Gemische derartiger Alkohole bevorzugt. Geeignet sind beispielsweise Methanol, Ethanol, n-Propanol, Isopropanol, n-Butanol, n-Hexanol, 2-Ethyl-1-hexanol, 1-Methoxy-2-propanol, Ethylenglykol, Propylenglykol, die isomeren Butandiole, Hexandiole oder Octandiole, Diethylenglykol, Dipropylenglykol, 2-Ethyl-1,3-hexandiol, 2,2,4-Trimethylpentandiol, Glycerin, Trimethylolpropan oder beliebige Gemische derartiger Alkohole.

Diese Co-Katalysatoren werden, falls überhaupt, in Mengen von bis zu 5, vorzugsweise von 0,5 bis 3 Gew.-%, bezogen auf das Ausgangsisocyanat, eingesetzt. Die Co-Katalysatoren reagieren mit dem Ausgangsdiisocyanat unter Urethanbildung. Diese Urethane stellen die eigentlichen Co-Katalysatoren dar. Hieraus folgt, daß prinzipiell anstelle der beispielhaft genannten Alkohole auch separat hergestellte Urethane als Co-Katalysatoren geeignet sind.

Zur Desaktivierung des Katalysators beim erfindungsgemäßen Verfahren sind grundsätzlich alle an sich bekannten Oxidationsmittel geeignet, mit denen die dreiwertigen Phosphorverbindungen, insbesondere die tertiären Phosphine in entsprechende fünfwertige Phosphorverbindungen, insbesondere die entsprechenden Phosphinoxide überführt werden können, wie sie z.B. in Houben-Weyl, Methoden der organischen Chemie, 4. Auflage, Band E2, Seite 41 bis 48, Band IV/1d, Seite 570 bis 574, Band XII/1, Seite 140 bis 144 und Band XII/2, Seite 109, 474, beschrieben sind, sofern sie bei ihrer erfindungsgemäßen Anwendung aufgrund ihrer Eigenfärbung nicht zu negativen Effekten auf die Farbqualität der erfindungsgemäßen Produkte führen.

Bevorzugt werden beim erfindungsgemäßen Verfahren organische Hydroperoxide, wie z.B. tert.-Butylhydroperoxid, Cumolhydroperoxid und Butanonperoxid, Dialkyl- bzw. Diacylperoxide, wie z.B. Diethylperoxid, Di-tert.-butylperoxid, Diamylperoxid, Dibenzoylperoxid und Dilauroylperoxid, Peroxycarbonsäuren, wie z.B. Peressigsäure, Perbenzoesäure, m-Chlorperbenzoesäure, sowie deren tert.-Butylester verwendet, aber auch Wasserstoffperoxid, Sauerstoff oder Luft, oder beliebige Mischungen solcher Oxidantien.

Im allgemeinen werden die obengenannten Oxidationsmittel, sofern sie in fester, flüssiger oder in ihrer handelsüblichen Form als Lösung vorliegen, in solchen Mengen zugegeben, daß auf jedes Mol des eingesetzten Katalysators mindestens 1 Mol und höchstens 3 Mole an "aktivem" Sauerstoff entfallen.

Bei der ebenfalls bevorzugten Verwendung von Sauerstoff oder Luft zur Desaktivierung des Katalysators leitet man eine der Katalysatormenge mindestens äquimolare, in der Regel aber vielfach molare Menge an trockenem Sauerstoff oder ein diese Menge an Sauerstoff enthaltendes Volumen Luft durch die Reaktionsmischung. Bei dieser Art der Reaktionsstoppung kann der Zusatz geringer Mengen eines Radikalstarters von Vorteil sein, wozu sich beispielsweise Verbindungen wie α,α'-Azo-isobutyronitril (AIBN) aber auch die obengenannten (Hydro)peroxide, wie z.B. Butanonperoxid, eignen. Bei Mitverwendung eines Radikalstarters wird dieser vor dem Einleiten von Sauerstoff oder Luft in Mengen von 50 bis 500 ppm, bezogen auf die gesamte Reaktionsmischung, eingesetzt.

Die Durchführung des erfindungsgemäßen Verfahrens erfolgt in an sich bekannter Weise, wie dies beispielsweise in den DE-OS'en 1 670 720, 1 954 093 oder 3 437 635 beschrieben ist.

Im allgemeinen wird so vorgegangen, daß man das Ausgangsdiisocyanat bzw. das Ausgangsdiisocyanatgemisch, gegebenenfalls unter Inertgasatmosphäre, bei einer Temperatur zwischen -20 und 100°C, bevorzugt 10 bis 80°C, mit einem erfindungsgemäß geeigneten Katalysator der beispielhaft genannten Art vermischt und die Oligomerisierungsreaktion innerhalb der genannten Temperaturbereiche ablaufen läßt.

Gegebenenfalls können hierbei, wie bereits erwähnt, dem Reaktionsgemisch vor oder während der Oligomerisierungsreaktion geeignete Co-Katalysatoren der beispielhaft genannten Art zugesetzt werden. Der Abbruch der Oligomerisierungsreaktion erfolgt nach Erreichen des angestrebten Oligomerisierungsgrads, der sich aus dem analytisch bestimmbaren NCO-Gehalt des Reaktionsgemischs errechnen läßt. Im allgemeinen wird die Oligomerisierungsreaktion bei Erreichen eines Oligomerisierungsgrads von 10 bis 60 %, vorzugsweise von 10 bis 50 % abgebrochen, wobei unter "Oligomerisierungsgrad" der Prozentsatz der im Ausgangsdiisocyanat vorliegenden Isocyanatgruppen zu verstehen ist, der während der Umsetzung unter Dimerisierung und/oder Trimerisierung sowie gegebenenfalls Urethanisierung aufgrund der Reaktion mit einem NCO-reaktiven Co-Katalysator abreagiert. Zum Abbruch der Oligomerisierungsreaktion werden dem Reaktionsgemisch feste oder flüssige Oxidationsmittel der beispielhaft genannten Art in Substanz oder gegebenenfalls in gelöster Form zugegeben und dieses anschließend über einen Zeitraum von 15 bis 120 Minuten auf eine Temperatur von 10 bis 100°C erwärmt. Gasförmige Oxidationsmittel (insbesondere Luft oder Sauerstoff) werden über einen entsprechenden Zeitraum durch die auf entsprechende Temperatur gebrachte Reaktionsmischung geleitet.

Im Anschluß an die erfindungsgemäße Abstoppung der Reaktion kann überschüssiges, nicht umgesetztes Diisocyanat durch an sich bekannte Methoden, wie z.B. Dünnschichtdestillation, vom erfindungsgemäßen Verfahrensprodukt abgetrennt und zur erneuten Verwendung eingesetzt werden. Lange Reaktionszeiten und hohe Temperaturen innerhalb der genannten Bereiche begünstigen im allgemeinen die Bildung von Trimeren (Isocyanuraten), während umgekehrt kurze Reaktionszeiten und vergleichsweise niedrige Temperaturen innerhalb der genannten Bereiche die Bildung von Dimeren (Uretdionen) begünstigen.

Das erfindungsgemäße Verfahren kann gegebenenfalls in Gegenwart von gegenüber Isocyanatgruppen inerten Lösungsmitteln durchgeführt werden. Geeignete Lösungsmittel sind beispielsweise Hexan, Toluol, Xylol, Chlorbenzol, Essigsäureethylester, Essigsäurebutylester, Ethylglykolacetat, Propylenglykolmonomethyletheracetat, Aceton, Methylisobutylketon, Methylenchlorid, N-Methylpyrrolidon oder beliebige Gemische derartiger Losungsmittel.

Die nach dem erfindungsgemäßen Verfahren aus den bevorzugt eingesetzten (cyclo)aliphatischen Diisocyanaten erhältlichen Produkte stellen, von überschüssigem, monomerem Ausgangsdiisocyanat befreit, bei Raumtemperatur teils feste, teils flüssige Substanzen dar. Während cycloaliphatische Diisocyanate, wie z.B. IPDI, vorzugsweise feste Verfahrensprodukte ergeben, werden ausgehend von aliphatischen Diisocyanaten, wie z.B. HDI, flüssige Produkte erhalten. Der Gehalt an monomerem Ausgangsdiisocyanat liegt i.a. unter 1 Gew-%, vorzugsweise unter 0,3 Gew.-%.

Besonders vorteilhaft erweist sich beim erfindungsgemäßen Verfahren die, aus der DE-OS 39 00 053 bekannte, farbaufhellende Wirkung anorganischer und organischer Peroxide auf modifizierte Polyisocyanate, die als Nebeneffekt auch bei der oxidativen Reaktionsstoppung eintritt. Daher weisen die nach dem erfindungsgemäßen Verfahren erhaltenen Produkte durchweg eine geringere Färbung aus als analog modifizierte Polyisocyanate, bei deren Herstellung Katalysatorgifte des Standes der Technik verwendet werden.

Bei Verwendung des als Ausgangsdiisocyanat besonders bevorzugt eingesetzten Hexamethylendiisocyanats oder Isophorondiisocyanats und des als Katalysator besonders bevorzugten Tri-n-octylphosphins (TOP), erhält man durch oxidative Reaktionsstoppung nach dem erfindungsgemäßen Verfahren ein Reaktionsgemisch, in dem nach gaschromatographischen Untersuchungen der Katalysator quantitativ in Tri-n-octylphosphinoxid (TOPO) überführt wurde.

Der im Vergleich zu den Siedepunkten von HDI und IPDI höher liegende Siedepunkt des TOPO gestattet es, bei Wahl geeigneter Destillationsbedingungen, nicht umgesetztes, überschüssiges Diisocyanat in reiner Form, d.h. ohne Verunreinigung durch desaktivierten Katalysator, zurückzugewinnen.

Wie gaschromatographische Untersuchungen und elementaranalytische Phosphorbestimmung der erfindungsgemäßen Verfahrensprodukte und der wiedergewonnenen Diisocyanate zeigen, verbleibt das Phosphinoxid vollständig im Destillationsrückstand, womit eine Anreicherung im Destillat, selbst bei mehrfacher Recyclisierung, ausgeschlossen ist.

Die erfindungsgemäßen Verfahrensprodukte verhalten sich hinsichtlich NCO-Gehalt, Viskosität und Farbqualität völlig lagerstabil. Sie stellen wertvolle Ausgangsmaterialien zur Herstellung von Polyurethankunststoffen nach dem Polyadditionsverfahren, insbesondere zur Herstellung von Ein- oder Zwei-Komponenten-Polyurethanlacken dar. Sie dienen in mit an sich bekannten Blockierungsmitteln blockierter Form als wertvolle Ausgangsmaterialien für Polyurethan-Einbrennlacke.

Bevorzugte Reaktionspartner für die erfindungsgemäßen, gegebenenfalls in blockierter Form vorliegenden Verfahrensprodukte bei der Herstellung von Polyurethanlacken sind die in der Polyurethanchemie an sich bekannten Polyhydroxypolyester und -ether, Polyhydroxypolyacrylate, Polycarbonsäuren und gegebenenfalls niedermolekulare, mehrwertige Alkohole. Auch Polyamine, insbesondere in blockierter Form als Polyketimine oder Oxazolidine sind brauchbare Reaktionspartner für die erfindungsgemäßen Mischpolymerisate.

Die Mengenverhältnisse werden im allgemeinen so gewählt, daß auf eine, gegebenenfalls blockierte, Isocyanatgruppe 0,8 bis 3, vorzugsweise 0,9 bis 1,1 Hydroxy-, Amino- und/oder Carboxylgruppen entfallen.

Zur Beschleunigung der Aushärtung können in bekannter Weise die in der Isocyanatchemie üblichen Katalysatoren verwendet werden, wie z.B. tert. Amine wie Triethylamin, Pyridin, Methylpyridin, Benzyldimethylamin, N,N-Endoethylenpiperazin, N-Methylpiperidin, Pentamethyldiethylentriamin, N,N-Dimethylaminocyclohexan, N,N'-Dimethylpiperazin usw., Metallsalze wie Eisen(III)chlorid, Zinkchlorid, Zink-2-ethylcaproat, Zinn(II)ethylcaproat, Dibutylzinn(IV)-dilaurat, Molybdänglykolat, usw..

Bei Verwendung der erfindungsgemäßen Verbindung bzw. Gemische in Einbrennlacken werden deren NCO-Gruppen ganz oder teilweise in bekannter Weise blockiert. Hierzu wird das Polyisocyanat mit einem geeigneten Blockierungsmittel, vorzugsweise bei erhöhter Temperatur, gegebenenfalls in Gegenwart eines geeigneten Katalysators (s.o.) umgesetzt.

Geeignete Blockierungsmittel sind beispielsweise Monophenole (Phenol, Kresole), tertiäre Alkohole (t.-Butanol, Dimethylphenylcarbinol), leicht enolisierbare Verbindungen (Acetessigester, Malonsäurederivate), sekundäre, aromatische Amine (N-Methylanilin, N-Phenylxylidin), Imide (Succinimid), Lactame (ε-Caprolactam, δ-Valerolactam), Oxime (Butanonoxim, Cyclohexanonoxim), Mercaptane (Methylmercaptan, Ethylmercaptan), Triazole (1H-1,2,4-triazol).

Zur Herstellung der Lackbindemittel werden beispielsweise gegebenenfalls blockiertes Polyisocyanat, polyfunktionelle Reaktionspartner, Katalysator und gegebenenfalls die üblichen Zusätze, wie z.B. Pigmente, Füll- und Farbstoffe und Verlaufmittel miteinander auf einem üblichen Mischaggregat, wie z.B. auf einer Sandmühle entweder mit oder ohne Lösungs- und Verdünnungsmittel gut vermischt und homogenisiert.

Als Lösungsmittel geeignet sind alle an sich bekannten üblichen Lacklösemittel wie z.B. Ethylacetat, Butylacetat, Ethylenglykolmonomethyl- oder ethyletheracetat, 1-Methoxypropyl-2-acetat, 2-Butanon, 4-Methyl-2-pentanon, Cyclohexanon, Toluol, Xylol, Solventnaphtha oder deren Gemische. Ebenfalls geeignet sind jedoch auch Lösemittel wie N-Methylpyrrolidon oder N-Methylcaprolactam oder Weichmacher wie z.B. solche auf Basis von Phosphorsäure-, Sulfonsäure- oder Phthalsäureester.

Die Anstrich- und Überzugsmittel können in Lösung oder aus der Schmelze oder in fester Form nach den üblichen Methoden wie z.B. Streichen, Rollen, Gießen, Spritzen, dem Wirbelsinterverfahren oder dem elektrostatischen Pulversprühverfahren auf den zu beschichtenden Gegenstand aufgebracht werden.

Die nachfolgenden Beispiele dienen der weiteren Erläuterung der Erfindung. Alle Prozentangaben beziehen sich, soweit nichts anderes vermerkt, auf Gewichtsprozente.

### Beispiele

### Beispiel 1

1500 g HDI werden bei 50°C nacheinander mit 15 g 2,2,4-Trimethyl-1,3-pentandiol (TMPD) und 4,5 g Tri-n-octylphosphin (TOP) versetzt und anschließend auf 60°C erwärmt. Nach 5 h Reaktionszeit beträgt der NCO-Gehalt der Mischung 41,2 %. Durch Zugabe von 2,6 g Butanonperoxid (50 %ig in Dimethylphthalat) und einstündiges Erwärmen auf 80°C wird die Reaktion abgebrochen. Der NCO-Gehalt der Rohware beträgt anschließend - ebenso wie nach 2-tägiger Lagerung - 40,8 %. Nach Abtrennen des nicht umgesetzten, monomeren Diisocyanates durch Dünnschichtdestillation (140°C, 0,05 mbar) erhält man 485 g eines schwach gelben Produktes mit einem NCO-Gehalt von 21,2 %, einer Viskosität von 180 mPa.s (23°C) und einem Restgehalt an monomeren HDI von 0,3 %. Der durch Heißtitration (3 h/180°C in o-Dichlorbenzol) ermittelte Gehalt an Uretdiongruppen (berechnet als C₂N₂O₂, Molekulargewicht = 84) beträgt 15,3 %. Das IR-Spektrum zeigt außerdem die Anwesenheit von Isocyanuratgruppen (1689, 1465 cm⁻¹) an.

Das zurückgewonnene Destillat enthält laut Analyse weniger als 1 ppm Phosphor.

Weder Harz noch Destillat zeigen bei 8-wöchiger Lagerung einen Abfall des NCO-Gehaltes.

### Beispiel 2

1000 g HDI werden bei 50° C mit 10 g 2,2,4-Trimethyl-1,3-pentandiol (TMPD) umgesetzt. Nachdem der NCO-Gehalt der Mischung auf 48,9 % abgesunken ist, gibt man 3,0 g Tri-n-octylphosphin (TOP) zu und erwärmt die Mischung auf 60°C. Nach 5 h 30 min beträgt der NCO-Gehalt der Mischung 42,5 %. Zur Reaktionsabstoppung gibt man 100 mg α,α'-Azo-isobutyrnitril (AIBN) zu, erwärmt auf 80°C und leitet 1 h lang einen trockenen Sauerstoffstrom (ca. 15 l/h) durch die Mischung. Nach Abkühlen auf Raumtemperatur beträgt der NCO-Gehalt - ebenso wie nach eintägiger Lagerung - 42,5 %. Die überschüssige Menge an nicht umgesetztem, monomerem Diisocyanat wird bei 130°C und einem Druck von 0,05 mbar durch Dünnschichtdestillation entfernt und man erhält 246 g eines schwach gelben Produktes mit einem NCO-Gehalt von 21,5 %, einer Viskosität von 150 mPa.s (23°C) und einem Gehalt an monomerem HDI von 0,3 %. Der durch Heißtitration (3 h/180°C in o-Dichlorbenzol) ermittelte Gehalt an Uretdiongruppen (berechnet als C₂N₂O₂, Molekulargewicht = 84) beträgt 17,2 %.

Das zurückgewonnene Destillat enhält laut Elementaranalyse keinen Phosphor. Harz und Destillat zeigen bei 4-wöchiger Lagerung keinen Abfall des NCO-Gehaltes.

### Beispiel 3

1000 g HDI werden bei 50°C nacheinander mit 10 g TMPD und 3 g TOP versetzt und anschließend für 7 h auf 60°C erwärmt. Die Reaktionsmischung weist nun einen NCO-Gehalt von 40,6 % auf. Durch Zugabe von 5 ml t-Butylhydroperoxid (3 M in iso-Octan) und einstündiges Erwärmen auf 80°C wird die Oligomerisierungsreaktion abgebrochen. Der NCO-Gehalt der Rohware beträgt 40,4 %. Durch Dünnschichtdestillation bei 130°C und einem Druck von 0,05 mbar wird das überschüssige, nicht umgesetzte HDI abgetrennt und man erhält 303 g eines schwach gelben Produkts mit einem NCO-Gehalt von 21,2 %, einer Viskosität von 300 mPa.s (23°C) und einem Restgehalt an monomeren HDI von 0,1 %. Der durch Heißtitration (3 h/180°C in o-Dichlorbenzol) ermittelte Gehalt an Uretdiongruppen (berechnet als C₂N₂O₂) beträgt 16,5 %. Das zurückgewonnene Destillat enthält laut Analyse weniger als 1 ppm an Phosphor. Weder Harz noch Destillat zeigen bei 4-wöchiger Lagerung einen Abfall des NCO-Gehaltes.

### Beispiel 4

1000 g HDI werden bei 50°C mit 1 g TMPD und 0,3 g TOP versetzt und anschließend 6 h bei 60°C gerührt, wobei der NCO-Gehalt der Mischung auf 41,3 % absinkt. Man gibt 0,5 ml einer 2-molaren Lösung von tert.-Butylhydroperoxid in iso-Octan zu und erwärmt 1 h auf 80°C. Nach Abkühlen auf RT beträgt der NCO-Gehalt der Mischung 40,4 % ebenso wie nach 30-tägiger Lagerung.

### Beispiel 5

200 g HDI werden bei 50°C mit 2 g TMPD und 0,6 g TOP versetzt. Nach 3,5 h bei 60°C ist der NCO-Gehalt der Mischung auf 43,8 % abgesunken. Nach Zugabe von 0,35 g Butanonperoxid (50 %ig in Dimethylphthalat) und einstündigem Erwärmen auf 80°C weist die Reaktionsmischung einen NCO-Gehalt von 43,7 % auf, der bei RT-Lagerung über 30 d konstant bleibt.

## Patentansprüche

1. Verfahren zur Herstellung von Uretdion- und/oder Isocyanuratgruppen aufweisenden Polyisocyanaten durch Oligomerisierung eines Teils der Isocyanatgruppen von organischen Diisocyanaten unter Bildung von Uretdion- und/oder Isocyanuratgruppen in Gegenwart von dreiwertigen Phosphor enthaltenden Katalysatoren, Abbruch der Oligomerisierungsreaktion bei einem gewünschten Oligomerisierungsgrad und anschließende Entfernung des nicht umgesetzten Diisocyanatüberschusses durch Dünnschichtdestillation, dadurch gekennzeichnet, daß man die Reaktion mit Hilfe eines geeigneten Oxidationsmittels unter Umwandlung des Katalysators in eine katalytisch inaktive, oxidierte Form abbricht.

2. Verfahren gemäß Anspruch 1, dadurch gekennzeichnet, daß man die Oligomerisierungsreaktion in Gegenwart von organischen Verbindungen, die mindestens ein an Sauerstoff, Stickstoff oder Schwefel gebundenes Wasserstoffatom und einen pKₛ-Wert von mindestens 6 aufweisen, als Co-Katalysator durchführt.

3. Verfahren gemäß Anspruch 1 und 2, dadurch gekennzeichnet, daß man als Katalysatoren tertiäre organische Phosphine verwendet.

4. Verfahren gemäß Anspruch 1 bis 3, dadurch gekennzeichnet, daß man als Katalysator Tri-n-octylphosphin verwendet.

5. Verfahren gemäß Anspruch 1 bis 4, dadurch gekennzeichnet, daß man als Oxidationsmittel (Luft-)-Sauerstoff und/oder organische Peroxide oder Gemische organischer Peroxide einsetzt.

6. Verfahren gemäß Anspruch 1 bis 5, dadurch gekennzeichnet, daß man als Ausgangsdiisocyanat ein (cyclo)aliphatisches Diisocyanat mit 6 bis 15 C-Atomen oder ein beliebiges Gemisch solcher Diisocyanate einsetzt.

7. Verfahren gemäß Anspruch 1 bis 6, dadurch gekennzeichnet, daß man als Ausgangsdiisocyanat 1,6-Diisocyanatohexan und/oder 1-Isocyanato-3,3,5-trimethyl-5-isocyanatomethylcyclohexan einsetzt.

## Claims

1. A process for the production of polyisocyanates containing uretdione and/or isocyanurate groups by partial oligomerization of the isocyanate groups of organic diisocyanates with formation of uretdione and/or isocyanurate groups in the presence of catalysts containing trivalent phosphorus, termination of the oligomerization reaction at a desired degree of oligomerization and subsequent removal of the unreacted diisocyanate excess by thin-layer distillation, characterized in that the reaction is terminated by a suitable oxidizing agent with conversion of the catalyst into a catalytically inactive oxidized form.

2. A process as claimed in claim 1, characterized in that the oligomerization reaction is carried out in the presence of organic compounds which contain at least one hydrogen atom attached to oxygen, nitrogen or sulfur and which have a pKₛ value of at least 6 as co-catalyst.

3. A process as claimed in claims 1 and 2, characterized in that tertiary organic phosphines are used as the catalysts.

4. A process as claimed in any of claims 1 to 3, characterized in that tri-n-octyl phosphine is used as the catalyst.

5. A process as claimed in claims 1 to 4, characterized in that (atmospheric) oxygen and/or organic peroxides or mixtures of organic peroxides is/are used as the oxidizing agent.

6. A process as claimed in claims 1 to 5, characterized in that a (cyclo)aliphatic diisocyanate containing 6 to 15 carbon atoms or a mixture of such diisocyanates is used as the starting diisocyanate.

7. A process as claimed in claims 1 to 6, characterized in that 1,6-diisocyanatohexane and/or 1-isocyanato-3,3,5-trimethyl-5-isocyanatomethyl cyclohexane is used as the starting diisocyanate.

## Revendications

1. Procédé de fabrication de polyisocyanates portant des groupes uretdione et/ou isocyanurate par oligomérisation d'une partie des groupes isocyanate de diisocyanates organiques avec formation de groupes uretdione et/ou isocyanurate en présence de catalyseurs contenant du phosphore trivalent, interruption de la réaction d'oligomérisation à un degré d'oligomérisation recherché et élimination subséquente de l'excédent de diisocyanates non convertis par distillation en couche mince, caractérisé en ce que la réaction est interrompue à l'aide d'un agent d'oxydation approprié avec transformation du catalyseur en une forme oxydée catalytiquement inactive.

2. Procédé selon la revendication 1, caractérisé en ce que la réaction d'oligomérisation se déroule en présence de composés organiques utilisés comme cocatalyseurs qui présentent au moins un atome d'hydrogène lié à de l'oxygène, de l'azote ou du soufre et une valeur de pK_{A} d'au moins 6.

3. Procédé selon les revendications 1 et 2, caractérisé en ce que les catalyseurs employés sont des phosphines organiques tertiaires.

4. Procédé selon les revendications 1 à 3, caractérisé en ce que le catalyseur utilisé est la tri-n-octyl-phosphine.

5. Procédé selon les revendications 1 à 4, caractérisé en ce que les agents d'oxydation employés sont l'oxygène (atmosphérique) et/ou des peroxydes organiques ou des mélanges de peroxydes organiques.

6. Procédé selon les revendications 1 à 5, caractérisé en ce que le diisocyanate initial employé est un diisocyanate (cyclo)aliphatique comprenant 6 à 15 atomes de C ou un mélange quelconque de ces diisocyanates.

7. Procédé selon les revendications 1 à 6, caractérisé en ce que le diisocyanate initial employé est le 1,6-diisocyanatohexane et/ou le 1-isocyanato-3,3,5-triméthyl-5-isocyanatométhylcyclohexane.
